# EUROPEAN PATENT APPLICATION

(11) **EP 4 160 484 A1**
(43) Date of publication of application: **05.04.2023**
(21) Application number: 21306379.5
(22) Date of filing: 01.10.2021
(51) Int. Cl.: G06N 3/063, G06N 3/04

(54) **« IN MATERIO COMPUTING » CLASSIFICATION DEVICE**

(71) Applicant: Centre national de la recherche scientifique, 75016 Paris (FR); Université Grenoble Alpes, 38400 Saint-Martin-d'Hères (FR); Forschungszentrum Jülich GmbH, 52425 Jülich (DE); Commissariat à l'Energie Atomique et aux Energies Alternatives, 75015 Paris (FR)
(72) Inventor: GAUDIN, Gilles, 38700 CORENC (FR); MIRON, Ioan Mihai, 38000 GRENOBLE (FR); YANG, Haozhe, 20014 SAN SEBASTIAN (ES); VATAJELU, Ioana, 38950 SAINT MARTIN LE VINOUX (FR); PINNA, Daniele, 40547 DUSSELDORF (DE)
(74) Representative: Lavoix

(57) **Abstract**

This device (1), comprising a pre-processing stage (10), a material system (20) and an identification stage (30), is characterized in that: the pre-processing stage (10) transforms a signal of interest (S₀) into an excitation signal (Sₑₓₜ); the material system (20) comprises a magnetic medium (50) subdivided into a plurality of zones (51) either in a first magnetic state or in a second magnetic state, an excitation module (26) generating, from the excitation signal (Sₑₓₜ), a physical excitation quantity adapted to displace the magnetic domain walls (56) in the magnetic medium (50), and a reading module (62) measuring the final magnetic configuration reached by the magnetic medium (50); and the identification stage (30) determines a class (Cᵢ) to which the signal of interest (S₀) belongs from the final magnetic configuration.

## Description

The present invention relates to a classification device of the "in materio computing" type.

The term "in materio computing" refers to an approach based on the use of a material system to replicate the operation of an artificial neural network.

An "in materio computing" device consists of a material system, between an input layer of neurons and an output layer of.

The input layer is a layer for pre-processing the signal of interest (carrying the information to be processed), in order to obtain a plurality of input data.

This input data is applied to the material system, where it is transformed according to a spatio-temporal process.

The state of the material system at the end of this spatio-temporal process is analysed by the output neuron layer, in order to label the signal of interest.

The material system must be large, i.e. it must have a state space whose dimensionality is greater than the characteristic size of the information to be processed, the aim being that the material system exhibits a separating power between signals of interest that is suitable for the identification task that the classification device must perform.

Such a material system must have other characteristics such as a short-term memory and a local non-linear response to the input data.

Such a material system behaves like an artificial neural network whose synaptic weights do not have to be determined by training. The synaptic links and their respective synaptic weights are randomly distributed. If the input and output neural networks consist of only one layer, only the determination of the synaptic weights of the links between the material system and the output layer requires training. But this determination is done by solving a linear system of equations, which does not require more computing power than a personal computer.

Another advantage of not having to train the material system as such is that the device can be used for different tasks. It is sufficient to identify the optimal pre-processing that the input neuron layer needs to perform to inject the information to be processed in a way that is suitable for the new task. It is also necessary to recalculate the synaptic weights of the links between the material system and the output neuron layer for each new task. The output neuron layer must therefore be trained with signals of interest labelled according to the new task to be performed.

The "in materio computing" approach allows to implement various neuromimetic processes such as neuronal dynamics, synapse plasticity,... A review of some of these implementations can be found in the paper D. Pryczyna, P. Zawal, T. Mazur, M. Strzelecki, P. L. Gentili & K. Szacilowski, Japanese Journal of Applied Physics 59, 050504 (2020).

This approach can thus enable the imitation of various artificial neural systems such as "physical reservoir computing" systems as described in the paper by M. Dale, J.F. Miller. & S. Stepney, "Reservoir Computing as a Model for In-Materio Computing", Chapter 22, Advances in Unconventional Computing, Volume 1, Theory 22, (Springer International Publishing, 2017) or in the article G. Tanaka et al. "Recent advances in physical reservoir computing: A review", Neural Networks 115, 100-123 (2019) or in the article K. Nakajima, "Physical reservoir computing - an introductory perspective", J. Appl. Phys. 59, (2020).

Numerous tests have been carried out in different fields of physics (photonic systems, mechanics, etc.), biology or chemistry, which can be found in the articles cited above.

However, to date, there is only one example of experimental success in a material system in the field of spin electronics, in the case of human speech recognition. This experiment, presented in the paper J. Torrejon, et al. "Neuromorphic computing with nanoscale spintronic oscillators", Nature 547, 428-431 (2017), uses a spin transfer oscillator.

However, this type of oscillator requires very elaborate nanofabrication techniques, since it uses a pillar with a diameter of less than 100 nm that is connected to each of its ends, with the pillar comprising a stack of a large number of magnetic layers.

Furthermore, the experiment presented in this article requires, as an input, in addition to the pre-processing step, a multiplexing step, so as to compensate for the loss of spatial parallelization resulting from the use of a single oscillator.

The aim of the present invention is to propose a device of the "in materio computing" type which is simple to implement.

To this end, the invention relates to a device according to the appended claims.

The invention and its advantages will be better understood on reading the following detailed description of two embodiments, given only as a non-limiting example, this description being made with reference to the appended drawings in which:
- Figure 1 is a schematic representation of a first embodiment of a classification device according to the invention;
- Figure 2 is a schematic representation, in top view, of the inhomogeneous magnetic medium of the device of figure 1;
- Figure 3 is a schematic top view of the inhomogeneous magnetic medium of Figure 2, the medium being in a predefined initial magnetic configuration;
- Figure 4 is a schematic top view of the inhomogeneous magnetic medium of Figure 2, the medium being in a particular final magnetic configuration;
- Figure 5 is a block diagram of a method of using the device of Figure 1; and
- Figure 6 is a schematic top view representation of the inhomogeneous magnetic medium of a second embodiment of the classification device according to the invention.

### General points

The classification device according to the invention is based on the displacement of magnetic domain walls in a one- or multi-dimensional inhomogeneous magnetic medium.

The inhomogeneous magnetic medium exhibits local variations in some of its physical parameters, so as to influence the displacement of the magnetic domain walls. The magnitude, position and nature of these variations may be natural and/or purposely chosen.

The inhomogeneous magnetic medium is first placed in a predefined initial magnetic configuration, that is common to all the classification operations associated with one same recognition task.

The inhomogeneous magnetic medium is then subjected, for a certain time interval, to a physical excitation quantity encoding the signal of interest to be classified. The physical excitation quantity is suitable for forcing the displacement of the magnetic domain walls.

The final magnetic configuration of the inhomogeneous magnetic medium, which is specific of the signal of interest applied, is analysed in order to be able to assign a label to said signal of interest.

### First embodiment

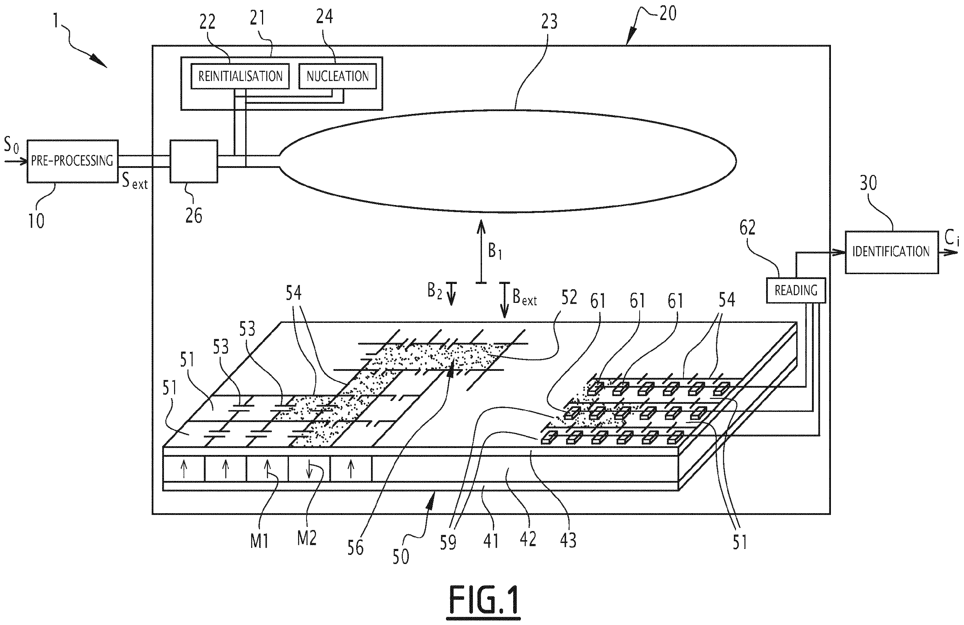

Figure 1 shows a first embodiment of a classification device according to the invention.

This device 1 comprises: a pre-processing input stage 10, transforming the signal of interest S₀ into an excitation signal Sₑₓₜ; an identification output stage 30; and, a material system 20 interposed between the pre-processing input stage and the identification output stage.

The material system comprises an inhomogeneous magnetic medium subdivided into a plurality of zones, each zone being in magnetic communication with at least one other zone. Each zone is capable of being either in a first magnetic state, in which a magnetisation of the zone in question is oriented along a predefined reference direction, or in a second magnetic state, in which the magnetisation of said zone is parallel to a direction opposite to the predefined reference direction

More specifically, in the first embodiment of Figures 1 to 4, the inhomogeneous magnetic medium 50 is a two-dimensional medium, for example of rectangular shape. Its thickness is much smaller than its characteristic length and width. Alternatively, other geometries than rectangular are possible.

The inhomogeneous magnetic medium 50 preferably consists of the stacking of a plurality of layers, at least one layer of which is made of a magnetic material whose magnetisation can be modified. For example, the inhomogeneous magnetic medium 50 comprises three layers 41, 42 and 43, for example of Pt, Co and Pt.

For example, the magnetisation of the intermediate layer is oriented along a reference direction that is perpendicular to the plane of the layer.

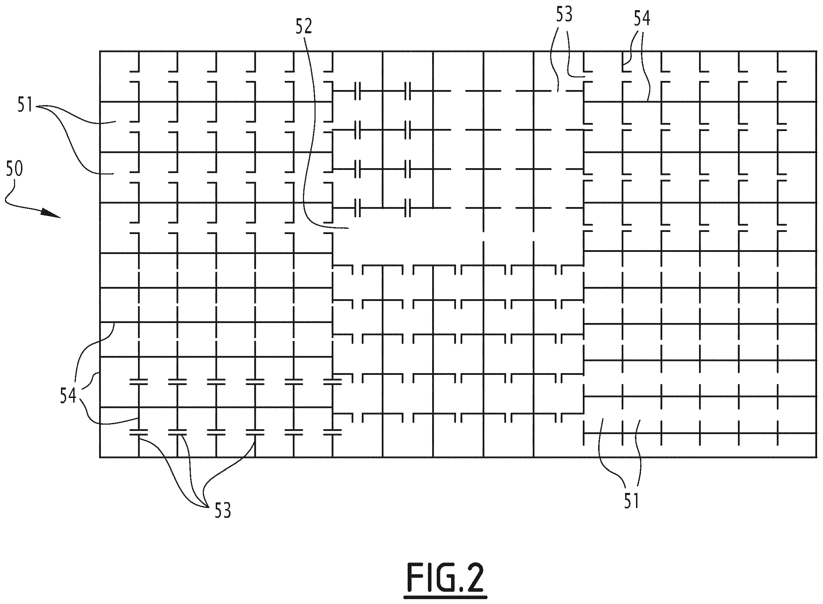

In the first embodiment, more clearly shown in Figure 2, a zone is constituted by a chamber 51. Thus, a plurality of chambers 51 is defined in the inhomogeneous magnetic medium 50.

A chamber 51 is bounded by walls 54 and has at least one opening 53 for the interconnection with an adjacent chamber. The opening is arranged through one of the walls of the chamber and allows movement of the magnetic domain walls between the exterior and interior of the chamber.

The chambers may have different geometries. In Figure 2, the chambers 51 are rectangular, but have varying lengths and/or widths. Alternatively, other geometries are possible, such as chambers with n sides (n greater than or equal to 3 and typically less than 10).

In Figure 2, the interconnection openings 53 between neighbouring chambers take the form of corridors, arranged parallel to the sides of the inhomogeneous magnetic medium 50, and having various lengths, widths and positions on a wall. Alternatively, the openings may take other forms.

In the first embodiment, any chamber 51 is accessible from a chamber of the medium 50, called source chamber 52, by one or more paths through a variable number of intermediate chambers (this number may be zero).

As they move under the effect of the excitation quantity, the magnetic domain walls propagate along one or more of these paths.

A final magnetic configuration is obtained after the application of the excitation quantity.

The chambers 51 are manufactured by locally modifying the magnetic material of the medium 50, so as to make it non-magnetic or by making it discontinuous, in order to delimit the walls 54 and the openings 53. This can be done, for example, by etching the magnetic material through a mask made of a resin or a sacrificial material (metals, dielectric, etc.), the design of the chambers having been previously defined in said mask by means of an optical or electronic lithography step, for example. Alternatively, the chambers 51 are made by means of a focused laser whose intensity is strong enough to make the magnetic material locally non-magnetic.

Each chamber 51 thus delimits a zone whose magnetisation can be oriented either in one direction along the reference direction, M1, the chamber then being in a first magnetic state, or in an opposite direction, M2, the chamber then being in a second magnetic state. In the first embodiment, the reference direction is perpendicular to the plane of the magnetic material. This is illustrated in the figures by a white box when the chamber is in the first state and a black box when the chamber is in the second state.

In this first embodiment, the chambers are adjacent to each other, so that the inhomogeneous magnetic medium is compact.

The material system 20 comprises a coil 23, as a means of generating a magnetic field. The coil 23 is arranged above the medium 50 in such a way that it can generate a substantially uniform magnetic field over the entire extent of the medium 50 and along the reference direction.

The material system 20 comprises an initialisation module 21, which, by supplying a suitable current to the coil 23, makes it possible to place the inhomogeneous magnetic medium 50 in a predefined initial magnetic configuration, so that all recognition operations start from an identical and known magnetic configuration.

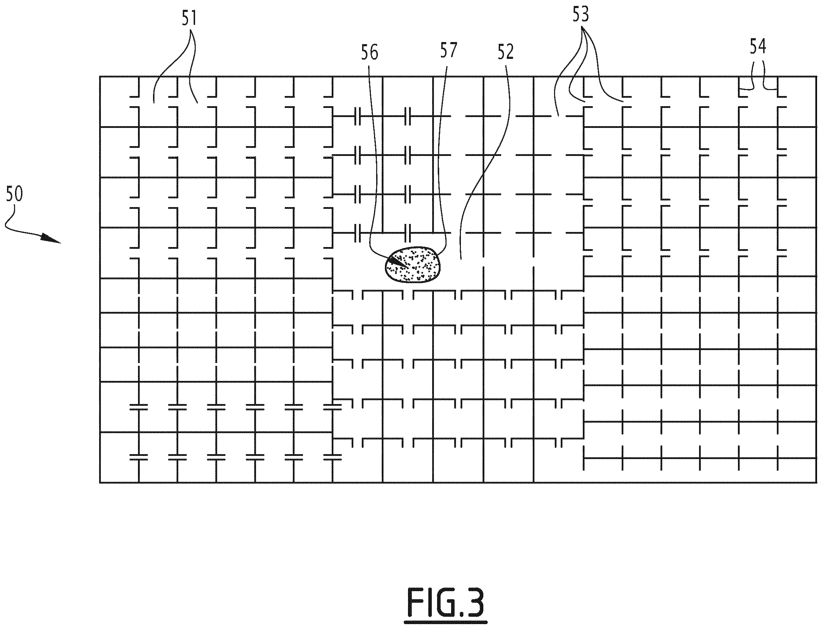

This initial magnetic configuration is for example defined as the configuration in which the plurality of chambers 51 are in the first magnetic state except for the source chamber 52, which is placed, at least partially, in the second magnetic state. This initial magnetic configuration is illustrated in Figure 3. The portion of the medium placed in the first magnetic state is shown in white while, while the portion of the medium placed in the second magnetic state is shown in black.

In the first embodiment, the initialization module 21 comprises, for example, a reset module 22 and a nucleation module 24, which are connected to the coil 23 so that it can be supplied with suitable currents.

To place the medium 50 in the initial magnetic configuration, a local defect is first induced in the magnetic material of the source chamber 52 to ensure the nucleation of a magnetic domain at this precise location when a second magnetic field B2 is applied.

The coil 23 is then energised by the nucleation module 24 so as to generate this second magnetic field B2. This field is anti-parallel to the reference direction and is locally sufficiently strong to switch the magnetisation of only a defined portion of the source chamber 52 to the second magnetic state M2.

Alternatively, the initialization module or the nucleation module is associated with a dedicated magnetic field generating device. For example, the nucleation module drives an electromagnet arranged directly above the source chamber in order to generate a second magnetic field suitable for nucleation when the reset of the inhomogeneous magnetic medium is required. The presence of a defect in the source chamber material is then no longer necessary.

Alternatively, all means known to the skilled person capable of modifying a magnetic configuration of the medium to return it to a predefined initial magnetic configuration and, if necessary, nucleate a domain of magnetisation in the source chamber, can be used. For example, instead of using a magnetic field, an electric field or an electric current could be used to reset the medium into the initial magnetic configuration.

Alternatively, the initial magnetic configuration is such that the medium has multiple source zones. If a nucleation module is implemented, then this nucleation module allows the different source zones to be placed, at least partially, in the second magnetic state. The nucleation module then comprises, for example, as many electromagnets arranged in the immediate vicinity of each of the source zones to generate a second magnetic field adapted to modify the magnetisation of a portion of each of the source zones, antiparallel to the reference direction. Alternatively, a local defect is induced in the magnetic material of each source zone to ensure that a magnetic domain is nucleated in each source zone upon the application of a second magnetic field, for example generated by means of an extended coil such as coil 23.

When powered by the reset module 22, the coil 23 generates a first magnetic field B1 parallel to the reference direction and sufficiently intense over the entire surface of the magnetic material to switch the magnetisation of each chamber 51 parallel to the reference direction, i.e. to place all the chambers in the first magnetic state M1. The reset module 22 thus puts the medium 50 in a uniform magnetic configuration.

The function of the nucleation module 24 is to nucleate a magnetic domain 56 within the magnetic material constituting the medium 50, the magnetisation of which is reversed with respect to the magnetisation of the uniform magnetic configuration adopted by the medium after the operation of the reset module 22.

The material system 20 comprises an excitation module 26 connected to the coil 23. The excitation module 26 supplies the coil 23, during a predefined time interval, with a current suitable to generate an excitation magnetic field Bₑₓₜ. The current produced by the module 26 is derived from the excitation signal Sₑₓₜ delivered by the pre-processing input stage 10.

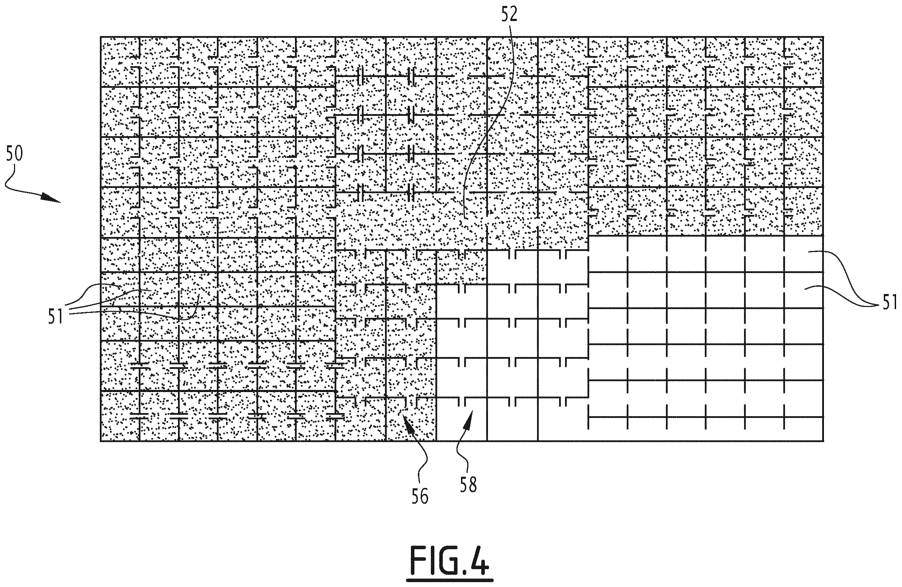

The excitation magnetic field Bext is capable of moving the magnetic domain walls, i.e. the interface 57 between a domain in the first magnetic state 58 and a domain in the second magnetic state 56 (Figure 4).

Alternatively, the excitation module is associated with a dedicated device for generating the physical excitation quantity, independent of the other components of the material system.

Alternatively, the excitation module is such that the physical excitation quantity is an electric field or an electric current. In the latter case, the current may be injected into the entire magnetic layer 42 from one of the edges of the medium. Alternatively, it may be injected into each of the source chambers (for example, by means of vias connected to transistors).

The material system 20 includes a readout module 62 measuring, after having applied the physical excitation quantity, the final magnetic state in which the inhomogeneous magnetic medium 50 is placed. The readout module 62 is, for example, associated with an array of magnetic sensors 61 (e.g. Hall effect sensors). Each sensor is located in close proximity to a corresponding chamber 51. It measures whether this chamber is in the first magnetic state M1 or in the second magnetic state M2.

The states of the chambers 51 form a pattern which is applied, by the readout module 62, to the identification layer 30.

The identification layer 30 preferably consists of an artificial neural network. The latter is configured to determine the class to which the signal of interest belongs among a plurality of predefined classes, from the final magnetic configuration that this signal of interest has conferred on the inhomogeneous magnetic medium. This artificial neural network can be simple ("perceptron") so as not to have to carry out complicated calculations, particularly in the learning phase. Alternatively, this artificial neural network comprises several layers.

### Method of use

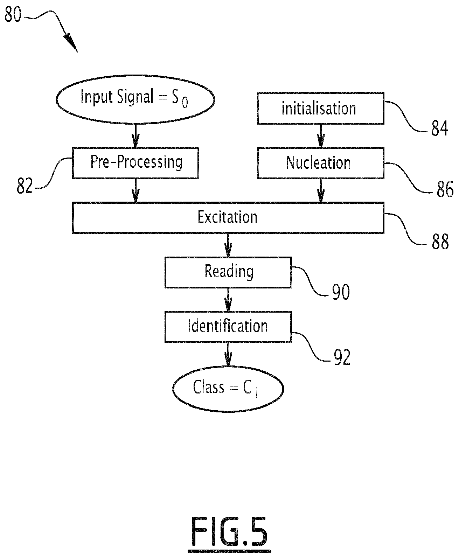

A method 80 of using the device of Figure 1 will be described with reference to Figure 5.

An inhomogeneous magnetic medium 50 is first prepared.

Prior to each recognition operation, the medium 50 is placed in the initial magnetic configuration using the initialisation means 21.

For example, in a step 84, the medium is first placed in a uniform magnetic configuration (all chambers 51 being then in the first magnetic state M1) by the application of a first magnetic field B₁ generated by the reset module 22.

Then, in a step 86, a magnetic domain 56 is nucleated in the source chamber 52 by the application of a second magnetic field B₂ generated by the nucleation module 24.

In a step 82, the signal of interest S₀ containing the information to be processed is pre-processed by the input stage 10 so as to obtain an excitation signal Sₑₓₜ.

Then, in step 88, an excitation magnetic field Bₑₓₜ is generated from the excitation signal Sₑₓₜ by the excitation module 26 and the coil 23. This excitation magnetic field Bₑₓₜ is applied to the medium 50 during a suitable time interval so as to allow the magnetic domain 56 to expand. In doing so, the medium 50 processes the information by changing the magnetisation state of certain its areas. The final magnetic configuration depends on the information contained in the signal of interest S₀.

Following step 88, the final magnetic configuration adopted by the medium 50 is read by the readout module 62.

Finally, the final magnetic configuration is analysed by the identification layer 30 to obtain a classification result for the information contained in the signal of interest S₀. The classification result is, for example, the class Ci to which the signal of interest belongs among a plurality of N predefined classes.

Insofar as the analysis algorithm implemented by the output stage is of the machine learning type, it is advisable, before using the recognition device 1, to determine the optimal parameterisation of the output stage 30.

For example, in a supervised learning approach, the device 1 is used on a database with labelled signals of interest. A labelled signal of interest is a signal of interest whose class is known. By means of a minimisation procedure, it is possible to determine the optimal values of the parameters of the output stage which allow the correct classification of the labelled signals processed.

### Experimental results

The device described above was used for a classification task consisting of recognising a digit in a speech signal as a signal of interest.

The signal of interest is extracted from the NIST TI-464 database. It is an electrical signal delivered by a microphone.

The pre-processing performed on the signal of interest is a frequency filtering in the time domain. The result of this pre-processing allows the excitation magnetic field to be modulated in time.

The class delivered by the output stage is the number recognised in the signal of interest among the possible values from 0 to 9.

Focusing on the female population, a recognition rate of 97% was achieved by using 66% of the signals of the database for training the output stage and 33% of the signals of the database for recognition operations. A recognition rate of 99% was obtained by using 80% of the signals of the database for training the output stage and 20% of the signals of the database for recognition operations.

These experimental results were obtained for an inhomogeneous Pt/Co/Pt magnetic medium with the following experimental conditions:
For the reset of the initial magnetic configuration, a first magnetic field with an amplitude between 10 mT and 100 mT was applied for a duration between 1 µs and 100 ms, preferably between 1 µs and 1 ms.

For the nucleation of a specific area of the medium, a defect was created by altering the magnetic properties of a source area through the use of a laser. As a result, the nucleation field is lower than the magnetic field required for switching the magnetisation of the unaltered magnetic material. The creation of a defect can also be done by ion irradiation or any other known means (lithography and etching, etc.).

The second magnetic field applied to nucleate the magnetic domain has an amplitude between 5 mT and 10 mT. This nucleating field is applied for a few milliseconds.

Once a domain has been nucleated, the second magnetic field is advantageously continued, but with a progressively decreasing amplitude, to increase the size of the magnetic domain until it fills the source chamber, but does not pass through the opening(s) of the source chamber. The second magnetic field then has an amplitude of 1 mT to 2 mT. It is applied for 100 ms.

For the excitation of the inhomogeneous magnetic medium, the excitation magnetic field has typically an amplitude between 1 mT and 10 mT. This amplitude is chosen to move the walls of the previously nucleated magnetic domain, while being insufficient to nucleate new magnetic domains of inverse magnetisation to that of the initial configuration.

In general, the duration of application of a magnetic field is related to its amplitude: the longer the duration of application, the lower the amplitude of the magnetic field must be to obtain the same effect.

The orders of magnitude given above are very dependent on the actual magnetic material used.

Tests are currently being carried out using an electric current rather than a magnetic field as the physical excitation quantity. The density of the electric current is typically between 10⁵ A/cm2 and 10⁸ A/cm2 and preferably between 10⁶ A/cm2 and 10⁷ A/cm2.

### Second embodiment

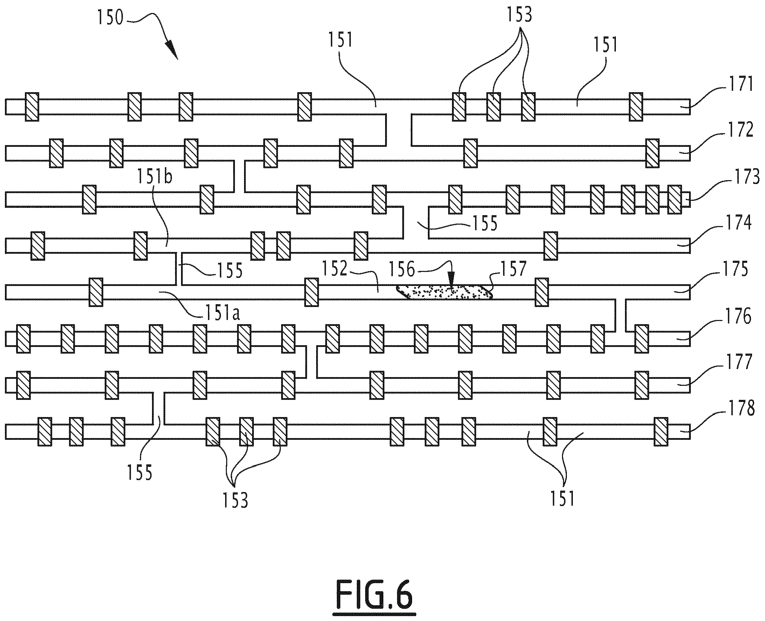

A second embodiment of an inhomogeneous magnetic medium is illustrated in Figure 6.

An element in Figure 6 which is similar to an element in Figure 1 is identified by a reference numeral which is increased by one hundred from the reference numeral used in Figure 1 to identify this similar element.

The inhomogeneous magnetic medium 150 consists of a plurality of magnetic wires 171 to 178.

Each magnetic wire is rectangular in cross-section. Alternatively, the wires have a different cross-sectional shape, such as circular or elliptical.

A magnetic wire may consist of a single material or alloy, or it may consist of several layers of different materials or alloys.

Advantageously, the magnetic wires are arranged parallel to each other in a plane. Alternatively, they are arranged parallel to each other in a volume so as to increase their density.

A magnetic wire has local magnetic defects 153. A defect is, for example, a local narrowing of the magnetic wire cross-section.

A section 151 of a magnetic wire between two consecutive defects 153 constitutes a zone of the inhomogeneous magnetic medium. A defect 153 thus constitutes a connection between two neighbouring zones.

In the embodiment shown in Figure 6, only one magnetic bridge 155 is provided between two neighbouring magnetic wires. A magnetic wire is connected with a neighbouring wire by any number of magnetic bridges, this number being possibly zero.

These wires thus form a medium for the propagation of magnetic domain walls from a source section as a source zone, such as the source section 152 of wire 175.

Alternatively, the medium may comprise a plurality of source sections. This is particularly the case when the wires are not connected to each other by one or more bridge(s).

Since a gap does exist between neighbouring magnetic wires, the medium according to this second embodiment is not compact, unlike in the first embodiment.

As in the first embodiment, in order to place the medium 150 in an initial magnetic configuration, either the magnetisation of medium is uniformly reset and then one or more domains is nucleated, or the magnetisation of the medium is initialized by moving back the magnetic domain walls to regain the initial magnetic configuration.

In the first case, for example, the first magnetic field may be applied by an electromagnet placed in the vicinity of the magnetic wires. The magnitude and duration of application of the first magnetic field is sufficient to put all sections of the magnetic wires into the first magnetic state. In a predetermined source section, a magnetic domain 156 is then nucleated by applying a second local magnetic field. This magnetic domain 156 is in a second magnetic state, that is opposite to the first magnetic state.

In the second case, there is simply an initialisation module to return to the initial magnetic configuration. A global magnetic field may be used whose amplitude and application time are not sufficient to switch all the magnetic wire sections to the first magnetic state, but sufficient to repel the magnetic domain walls and decrease the domain area until it is contained within the source section.

For the excitation of the medium, an excitation magnetic field is used. This field is, for example, time modulated with an amplitude varying between 1 and 10 mT. It allows the magnetic domain 156 in the second state of magnetisation and initially nucleated in the source section 152 to grow the domain 158 by moving its wall 157.

For reading the magnetic configuration of the inhomogeneous magnetic medium, as in the first embodiment, a readout module 62 may be used that is made of an array of sensors (for example magnetic sensors) placed in the vicinity of each section or groups of sections of magnetic wires of the medium.

Alternatively, magneto-optical imaging (wide-field microscopy using the Kerr effect) can be easily implemented.

In the case of unconnected magnetic wires that are made from layers deposited to create a GMR (Giant MagnetoResistive) or TMR (Tunnel Magnetoresistance) type stack, it is advantageous to measure the magnetoresistance of each magnetic wire. The measurement of the electrical resistance even allows access, in certain cases, to the position along a magnetic wire of the magnetic domain wall. The magnetic wire will then have a rectangular cross-section. It may also be circular in cross-section, made from layers wound on top of each other to create a GMR or TMR type stack.

### Alternative embodiments

Numerous alternative embodiments are conceivable, relating to the inhomogeneous magnetic medium for the propagation of the magnetic domain walls, the pre-processing and coding of the information to be processed and finally the analysis of the image of the magnetic domain walls in the final magnetic configuration.

As far as the propagation of the magnetic domain walls is concerned, the magnetic medium can be made to have alterations that influence the movement of the magnetic domain walls, both in terms of their dynamics and in terms of the paths they follow through the magnetic medium.

An alterations is a local variation of one or more physical parameters of the magnetic medium that influence the displacement of the magnetic domain walls. Examples of physical parameters are magnetic anisotropy, magnetic symmetric exchange, magnetic asymmetric exchange (also called Dzyaloshinkii - Moryia interaction), local magnetisation, dipole field, etc.

The alterations are intrinsic to the magnetic material or are deliberately made in the magnetic material.

The amplitude of the variations caused by these alterations and their spatial distribution must be such that the magnetic pattern resulting from the excitation of the magnetic medium by means of a first piece of information to be processed is as different as possible from that obtained by the excitation of the same magnetic medium by means of a second piece of information to be processed, when it is desired for the recognition device to be able to separate the first and second pieces of information from one another.

With regard to intrinsic alterations, it can be said that any magnetic material is nonuniform in the sense that it contains local variations of physical parameters. It is then sought that the action of these local variations is sufficiently large to influence the propagation of the magnetic domain walls. These alterations are caused, for example, by grain boundaries in polycrystalline materials, by macles in crystalline materials, or by local variations in the composition of the magnetic material in the case of alloys or local variations in thickness in the case where the magnetic material is obtained by the successive deposition of very thin magnetic layers. These intrinsic alterations can be controlled by the structure of the magnetic material itself, for example during the deposition of the magnetic layer: deposition parameters (residual pressure, pressure of the gas used for deposition, power on the targets, deposition speed, etc.); substrate on which the magnetic medium is deposited (amorphous substrate such as thermal SiO2 obtained on Silicon, crystalline substrate such as Si, MgO or AlOx, etc.) ...

Regarding permanent alterations, these can, for example, be carried out by local engineering of the magnetic material to modify the physical parameters mentioned above, even to the point of making the material non-magnetic. Various techniques can be envisaged: etching of the magnetic material by IBE ("Ion Beam Etching") or RIE ("Reactive-Ion Etching") after lithography steps and possibly a sacrificial deposition; use of a focused ion beam (FIB); use of a focused laser; local irradiation by a beam of light ions such as He; implementation of atoms; ...

An example of permanent alteration is the modification of the geometry of the magnetic medium. In the first embodiment, this involved delineating chambers and openings between these chambers to constrain the path of the magnetic domain walls. In the second embodiment, in which the medium consists of yarns, an alteration may consist of a local modification of the magnetic wire, for example the modification of its width (in particular a shrinkage of its diameter), of the composition of the material of the magnetic wire, or of the thicknesses of the layers making up the magnetic wire in the case of a multilayer material.

The spatial distribution of the zones may follow different patterns. A two dimensional magnetic medium can be structured with adjacent zones (this is the case of the chambers of the first embodiment). Another possibility is to have a magnetic medium with non-adjacent zones (this is the case of the wires of the second embodiment). In this case, the zones are preferably distributed regularly, for example in a periodic pattern.

The information to be processed is pre-processed in order to produce a modulated physical excitation quantity capable of displacing the magnetic domain walls. The modulation can be done in many different ways. For example, the information to be processed can be coded in frequency or time by any means known to the skilled person. More generally, various coding methods and associated pre-processing operations can be found in the literature.

This physical excitation quantity may be a magnetic field as in the first and second embodiments, or, alternatively, an electric field, an electric current, a spin current, or a mechanical or thermal stress applied to the magnetic medium. The modulation of this physical excitation quantity may be added to a static value of this quantity. Finally, these different physical excitation quantities can be combined to excite the medium. A modulated electric current can, for example, be injected in the presence of a modulated magnetic field or a modulated stress.

With respect to the nucleation of a domain having a different (preferably opposite) magnetisation to the magnetisation of the rest of the magnetic medium, the nucleation signal may be any physical quantity suitable for exciting magnetic domains in the medium, such as for example: a magnetic field with an orientation chosen in accordance with the direction of the magnetisation to be conferred to the nucleated domain of the medium; an electric current injected into the medium or into a layer adjacent to it, in any direction, perpendicular or parallel to the plane of the medium at the injection point; an electric field applied globally or locally, perpendicular to the plane of the medium at the injection point; a stress, for example mechanical or thermal, applied to the medium; etc. These types of nucleation signals can also be used in combination. For example, an electric field applied globally or locally, perpendicular to the plane of the medium at the injection point, with a magnetic field of selected orientation, or the same magnetic field but combined with a mechanical or thermal constraint applied to the medium.

Similarly, with respect to the reset, the reset signal can be any physical quantity that is capable of resetting the magnetizations of the different zones of the magnetic medium to a predefined magnetic configuration to obtain a uniform magnetic configuration.

Similarly, with respect to initialization, the initialization signal may be any physical quantity suitable to push the domain walls back to confine the domain(s) to the corresponding source zone(s).

Concerning the means for reading the final magnetic configuration of the magnetic medium, this may be optical means for acquiring an image of the medium. This can be done, for example, by means of Kerr effect microscopy. The field of observation can be wide or focused. In the latter case, a scanning technique is used to cover the entire surface of the magnetic medium. Another possibility is to detect the magnetic state of the medium by means of suitable local sensors as described above. A sensor can be, for example, a stack of the magnetotunnel resistance (TMR) or giant magnetoresistance (GMR) type, structured as a pillar and attached to the magnetic medium, in direct contact with this latter. Alternatively, the sensors are mounted on a substrate and this substrate is positioned close to the medium.

In this configuration, in order to further increase the space of possible states of the material system, several magnetic media can be stacked on top of each other. If a single sensor array is used for the entire stack, then the sensors will detect the cumulative effect of the different stacked magnetic media.

With regard to the analysis of the final magnetic configuration to perform the classification in itself, this analysis can implement any known algorithm such as for example a support vector machine or wide margin separator algorithm. The need to determine weights is again present, but when the output stage has only one layer of neurons, only linear equations are involved.

### Advantages

The recognition device according to the invention is a practical realisation of the concepts of "in materio computing", which is simple, integrable and compatible with CMOS (Complementary Metal-Oxide-Semiconductor) technology.

The recognition device according to the invention can perform a wide range of classification tasks, such as recognition, sorting, detection and prediction.

One recognition device can be easily configured to perform new tasks.

## Claims

1. A classification device (1) comprising a pre-processing stage (10), an identification stage (30), and a material system (20) interposed between the pre-processing stage and the identification stage, the classification device being **characterized in that**:
- the material system (20) comprises an inhomogeneous magnetic medium (50) subdivided into a plurality of zones (51), each zone being in magnetic communication, directly or indirectly via one or more intermediate zones, with at least one so-called source zone (52), each zone being either in a first magnetic state in which a magnetization (M1) of said zone is oriented in a first direction, or in a second magnetic state in which the magnetization (M2) of said zone is oriented in a second direction opposite to the first direction;
- the material system (20) comprises an initialization module (21) for placing the plurality of zones (51) of the inhomogeneous magnetic medium (50) in a predefined initial magnetic configuration, wherein the plurality of zones is in the first magnetic state with the exception of the source zone(s) (52), which are placed, at least partially, in the second magnetic state;
- the pre-processing stage (10) is adapted to transform a signal of interest (S₀) into an excitation signal (Sₑₓₜ);
- the material system (20) comprises an excitation module (26) adapted to generate, from the excitation signal (Sₑₓₜ), a physical excitation quantity adapted to excite the inhomogeneous magnetic medium (50) so as to displace magnetic domain walls (56), which are located at the interface between a portion of the inhomogeneous magnetic medium (50) placed in the first magnetic state and a portion of the inhomogeneous magnetic medium (50) placed in the second magnetic state, the excitation module (26) applying the physical excitation quantity during a predefined time interval, so as to increase the number of zones in the second magnetic state from the source zone(s) (52);
- the material system (20) comprises a readout module (62) adapted to measure, after application of the physical excitation quantity, the final magnetic configuration reached by the inhomogeneous magnetic medium (50), the final magnetic configuration being applied as an input to the identification stage (30); and
- the identification stage (30) is adapted to determine a class (Cᵢ) to which the signal of interest (S₀) belongs from a plurality of predetermined classes from the final magnetic configuration.

2. The device according to claim 1, wherein the initialisation module (21) allows the magnetic domain walls to be moved back to return the inhomogeneous magnetic medium (50) to the initial magnetic configuration.

3. A device according to claim 1 or claim 2, wherein the initialization module (21) comprises a reset module (22), for placing the plurality of zones (51) of the inhomogeneous magnetic medium (50) in the first magnetic state, and a nucleation module (24) for placing, at least partially, the source zone(s) (52) in the second magnetic state.

4. A device according to claim 3, wherein the nucleation module (24) is associated with a device for generating a local magnetic field (B₂), an intensity of which allows to switch, at least partially, the source zone(s) (52) from the first magnetic state to the second magnetic state.

5. A device according to any one of claims 1 to 4, wherein the inhomogeneous magnetic medium (50) comprises a plurality of chambers (51), each chamber being bounded by a plurality of walls (54) and having at least one opening (53) to a neighbouring chamber, an opening allowing the movement of the magnetic domain walls between said chamber and said neighbouring chamber, a chamber constituting one zone of the plurality of zones.

6. A device according to any one of claims 1 to 4, wherein the inhomogeneous magnetic medium (150) comprises a plurality of magnetic wires (171-178), each magnetic wire being subdivided into a plurality of sections (151), a section being separated from an adjacent section by a magnetic defect (153), a magnetic defect allowing the movement of the magnetic domain walls between said section and said adjacent section, a section constituting a zone of the plurality of zones.

7. A device according to any of the preceding claims, wherein the physical excitation quantity generated by the excitation module (26) is a magnetic field, an electric field, an electric current, or a combination thereof, modulated so as to encode the signal of interest (S₀).

8. A device according to claim 7, wherein the excitation module (26) energized an electromagnet or a coil adapted to generate an excitation magnetic field (Bₑₓₜ) over the whole inhomogeneous magnetic medium (50) as a physical excitation quantity.

9. A device according to any one of the preceding claims, wherein the identification stage (30) comprises an artificial neural network.

10. A device according to any one of the preceding claims, wherein the reading module (62) comprises a plurality of sensors, preferably magnetic sensors (61), arranged in the vicinity of the inhomogeneous magnetic medium (50), each sensor allowing to determine the magnetic state of one or more zone(s) (51) associated with said sensor.
